Europäisches Patentamt

European Patent Office

Office européen des brevets

(19)

(11) Veröffentlichungsnummer: **0 384 016**

**A1**

(12) **EUROPÄISCHE PATENTANMELDUNG**

(21) Anmeldenummer: 89122221.8

(22) Anmeldetag: 01.12.89

(51) Int. Cl.⁵: **B01D 61/36, B01D 61/58, B01D 53/22, C07C 29/76, //C12M1/12**

(30) Priorität: 01.12.88 DE 3840576

(43) Veröffentlichungstag der Anmeldung:
29.08.90 Patentblatt 90/35

(84) Benannte Vertragsstaaten:
AT BE CH DE ES FR GB GR IT LI LU NL SE

(71) Anmelder: ALUSINGEN GmbH
Alusingen Platz 1
D-7700 Singen/Htwl.(DE)

(72) Erfinder: Gudernatsch, Wilhelm, Dipl.-Ing.
Rosenbergstrasse 103

D-7000 Stuttgart 1(DE)
Erfinder: Chmiel, Horst, Prof. Dr.-Ing.
Paracelsusstrasse 14
D-7250 Leonberg(DE)
Erfinder: Strathmann, Heinrich, Prof. Dr.-Ing.
Milanweg 15
D-7400 Tübingen(DE)

(74) Vertreter: Finck, Dieter et al
Patentanwälte v. Füner, Ebbinghaus, Finck
Mariahilfplatz 2 & 3
D-8000 München 90(DE)

(54) **Verfahren und Vorrichtung zum Abtrennen von Komponenten aus einem Fluidgemisch.**

(57) Bei dem Verfahren zum Abtrennen wenigstens einer Komponente, insbesondere von Lösemitteln, aus einem Fluidgemisch wird das Fluidgemisch an einer Selektier-Membran (2) vorbeigeleitet und an dieser durch einen permeatseitigen Partialdruck, der niedriger ist als der des Fluidgemischs, in ein Permeat, dessen Konzentration an abzutrennender Komponente höher ist als die des Fluidgemischs, und in ein Retentat getrennt, dessen Konzentration an abzutrennender Komponente geringer ist als die des Fluidgemischs. Das Permeat wird aufgeheizt (5). Um die abzutrennenden Komponenten mit wenig Aufwand in konzentrierter Form zu gewinnen, wird das Permeat der Selektier-Membran (2) an einer Aufkonzentrier-Membran (7) mit einer bezüglich der Selektier-Membran (2) inversen Selektivität vorbeigeleitet. An der Aufkonzentrier-Membran (7) wird das Permeat durch einen endpermeatseitigen Partialdruck, der niedriger ist als der des vorbeigeleiteten Permeats, in ein Endpermeat, dessen Konzentration an abzutrennender Komponente geringer ist als die des Permeats der Selektier-Membran (2), und in ein Endretentat getrennt, dessen Konzentration an abzutrennender Komponente höher ist als die des Permeats der Selektier-Membran (2).

I. Stufe    II. Stufe

## Verfahren und Vorrichtung zum Abtrennen von Komponenten aus einem Fluidgemisch

Die Erfindung betrifft ein Verfahren zum Abtrennen von wenigstens einer Komponente, insbesondere von Lösemitteln, aus einem Fluidgemisch, bei dem das Fluidgemisch an einer Selektier-Membran vorbeigeleitet und an dieser durch einen permeatseitigen Partialdruck, der niedriger ist als der des Fluidgemisches, in ein Permeat, dessen Konzentration an abzutrennender Komponente höher ist als die des Fluidgemischs, und in ein Retentat getrennt wird, dessen Konzentration an abzutrennender Komponente geringer ist als die des Fluidgemischs, und bei dem wenigstens das Permeat aufgeheizt wird.

Die Erfindung betrifft außerdem eine Vorrichtung zur Durchführung dieses Verfahrens, mit einem Membranmodul, das durch eine für die abzutrennenden Komponenten durchlässigen Selektiermembran in einen Permeatbereich und einen Retentatbereich getrennt ist, der mit einer Fluidgemischzuführung und einer Retentatabführung in Verbindung steht, mit einer Pumpe, die einlaßseitig mit dem Permeatbereich für ein Abziehen von Permeat verbunden ist und mit einer Heizeinrichtung für wenigstens das Permeat. Lösemittel werden großtechnisch, beispielsweise als Verdünner (z. B. für Lacke) oder als Reinigungsmittel verwendet. Daneben dienen eine Reihe von Lösemitteln, wie beispielsweise Äthanol als Treibstoffzusatz.

Bisher werden Lösemittel überwiegend auf chemischem Wege hergestellt. Aus einer Reihe von Gründen von denen exemplarisch nur der Umweltschutz genannt werden soll, besteht der Bedarf, solche Lösemittel biotechnisch herzustellen. Bei der Herstellung von Lösemitteln durch Biokatalysatoren, wie beispielsweise Bakterien, Hefen, Enzymen im sogenannten Batch-Verfahren hemmt das produzierte Lösemittel mit zunehmender Konzentration die Produktivität des Biokatalysators. Mikroorganismen, wie Zymomonas mobiles, die zur Äthanol-Produktion verwendet werden, oder Acetobacter, die Aceton-Butanol-Produktion verwendet werden, stellen bei Erreichen einer Konzentration von etwa 6 bis 12% Lösemittel im Biofluid ihre Produktion ein.

Es wäre deshalb wünschenswert, bei der Herstellung von Lösemitteln mittels Biokatalysatoren das entstehende Lösemittel kontinuierlich aus dem Reaktor entfernen zu können.

Gattungsgemäße Verfahren und Vorrichtungen sind beispielsweise aus der PCT-Veröffentlichung WO 86/01425, den US-PSen 36 08 610 bzw. 36 24 683, sowie der DE-A 33 04 956 bekannt. Bei Verwendung dieser Vorrichtungen zum Trennen von Fluidgemischen fällt das Produkt in sehr geringer Konzentration in wässriger Lösung an. Die energetisch aufwendige Aufkonzentrierung verschlechtert die Wirtschaftlichkeit des Prozesses.

Darüber hinaus ist es mit den bekannten Vorrichtungen nicht möglich, beispielsweise direkt Bioalkohol als Treibstoffzusatz herzustellen, da hier normalerweise eine Äthanol-Konzentration von 99,9% gefordert wird. Dies gilt auch für die biotechnische Herstellung anderer Lösemittel, wie beispielsweise Aceton-Butanol.

Ähnliche Probleme treten bei der Abtrennung organischer Lösemittel aus Industrieabwässern, aus dem Grundwasser etc. auf.

An vielen Stellen, beispielsweise in der Nähe von Chemiewerken, von Deponien, etc. sind toxische Substanzen und insbesondere Lösemittel in das Grundwasser gelangt. Zur Entfernung dieser Substanzen werden in der Regel Bohrungen niedergebracht, über die das Grundwasser zur Reinigung an die Oberfläche und anschließend zurückgepumpt wird. Die Reinigung des Grundwassers mit bekannten Verfahren bzw. Vorrichtungen ist jedoch zeitaufwendig und damit teuer.

Darüber hinaus besitzen Trenn- und Aufkonzentriervorrichtungen, die direkt an die mit Lösemittel beladene Abluft und Abwässer erzeugenden Produktionseinheiten angekoppelt werden können, eine steigende Bedeutung, um gleichzeitig die kostensparende Rückführung der Lösemittel in den benötigten Prozeß sowie die Eliminierung eines gravierenden Umweltproblems zu gewährleisten.

Der Erfindung liegt deshalb die Aufgabe zugrunde, ein Verfahren sowie eine Vorrichtung zum Abtrennen von wenigstens einer Komponente aus einem Fluidgemisch zu schaffen, mit denen die abzutrennenden Komponenten mit wenig Aufwand in konzentrierter Form erhalten werden können.

Diese Aufgabe wird ausgehend vom gattungsgemäßen Stand der Technik verfahrensmäßig dadurch gelöst, daß das Permeat der Selektier-Membran an einer Aufkonzentrier-Membran mit einer bezüglich der Selektier-Membran inversen Selektivität vorbeigeleitet wird, an der es durch einen Endpermeatseitigen Partialdruck, der niedriger ist als der des vorbeigeleiteten Permeats in ein Endpermeat, dessen Konzentration an abzutrennender Komponente geringer ist als die des Permeats der Selektier-Membran und in ein Endretentat getrennt wird, dessen Konzentration an abzutrennender Komponente höher ist als die des Permeats der Selektier-Membran.

Es wurde nämlich erkannt, daß die Wirtschaftlichkeit des gattungsgemäßen Verfahren häufig dadurch beeinträchtigt wird, daß die abzutrennenden Komponenten, also ein oder mehrere Lösemittel vermischt mit weiteren Komponenten, insbesondere in wässriger Lösung vorliegen. Zur Trennung der abzutrennenden Komponenten von dem Permeat der Selektier-Membran verläßt die Erfindung das bisherige Konzept, gemäß dem mehrere gleichartige Membranen "hintereinander" geschaltet werden.

Statt dessen wird das Permeat der Selektier-Membran an einer inversen Membran, der Aufkonzentrier-Membran, vorbeigeleitet, d. h. einer Membran, deren Selektivität so gewählt ist, daß die abzutrennende Komponente bzw. die abzutrennenden Komponenten im Endretentatbereich der Aufkonzentrier-Membran verbleiben und die zusätzlich noch vorhandenen - eigentlich unerwünschten - Komponenten in den Endpermeatbereich der Aufkonzentrier-Membran gelangen.

Vorrichtungsmäßig wird die Aufgabe ausgehend vom gattungsgemäßen Stand der Technik dadurch gelöst, daß die Pumpe förderseitig mit einem Aufkonzentrier-Membranmodul verbunden ist, das durch wenigstens eine bezüglich der Selektier-Membran invers selektive Aufkonzentrier-Membran in einen Endretentatbereich und einen mit dem Einlaß einer weiteren Pumpe verbundenen Endpermeatbereich getrennt ist.

Die Selektier-Membran sollte eine hohe Permeabilität für organische Lösemittel aufweisen. Sie kann beispielsweise eine Porenmembran (Ultrafiltrationsmembran) sein. Vorteilhafterweise ist die Selektier-Membran jedoch eine Pervaporationsmembran, so daß das Permeat dampfförmig ist.

Dies ist besonders von Vorteil, wenn die abzutrennende Komponente ein organisches Lösemittel, beispielsweise Äthanol, chlorierte Kohlenwasserstoffe, Toluol, Xylol, Äther und Ester etc. und die zusätzliche Komponente Wasser ist. Für niedermolekulare Lösemittel durchlässige Perforationsmembrane weisen nämlich zwangsläufig auch eine gewisse Durchlässigkeit für $CO_2$ und $H_2O$ und $O_2$ auf. Die Selektiermembran sollte insbesondere dann, wenn die Vorrichtung zum kontinuierlichen Abziehen von Lösemitteln aus Bioreaktoren verwendet wird, für das Substrat, Meersalze und vor allem für Mikroorganismen vollständig undurchlässig sein.

Die Aufkonzentrier-Membran ist vorteilhafterweise eine Lösungsmittel-Diffusionsmembran. Dabei ist zu berücksichtigen, daß durch die Lösungs-Diffusionsmembran Wasser leicht hindurchtreten soll, während sie für das Lösemittel, das ein höheres Molekulargewicht hat, eine möglichst geringe Permeabilität haben soll. Dies bedeutet, daß für die Permeabilität der Aufkonzentrier-Membran ihre Diffusivität bestimmend sein muß.

Zweckmäßigerweise weist die Selektier-Membran eine selektive Schicht aus einem Elastomer und die Aufkonzentrier-Membran eine selektive Schicht aus einem glasartigen Polymer auf.

Der Diffusionskoeffizient nimmt in Elastomeren weniger stark mit dem Molekulargewicht der permeierenden Komponente ab als in glasartigem Polymer. Die Löslichkeit dagegen nimmt sowohl in einem Elastomer als auch in einem glasartigen Polymer mit zunehmendem Molekulargewicht des Permeanten zu. Damit nimmt die Permeabilität, die sowohl durch den Diffusivität- als auch durch den Löslichkeitskoeffizienten bestimmt wird, in glasartigen Polymeren mit zunehmendem Molekulargewicht des Permeats ab. In Elastomeren kann sie jedoch mit steigendem Molekulargewicht zunehmen, und zwar genau dann, wenn der Logarithmus des Diffusionskoeffizienten weniger stark abnimmt als der Logarithmus der Löslichkeit zunimmt.

Ein Beispiel für ein Elastomer der Selektier-Membran ist Polydimethylseloxan.

Es ist zweckmäßig, das Permeat der Selektier-Membran durch eine Heizeinrichtung zwischen dem Membranmodul und der Aufkonzentrier-Membran so aufzuheizen, daß es dampfförmig an der Aufkonzentrier-Membran vorbeigeleitet wird. Auf diese Weise fällt eine "Zwischen"-Kondensation des Permeats weg, wodurch die Wirtschaftlichkeit des Prozesses wesentlich erhöht wird.

Der Permeat-Dampfstrom kann beispielsweise durch einen Wärmetauscher strömen, der ihn weiter aufheizt. Besonders bevorzugt ist es jedoch, wenn die Heizeinrichtung und die Aufkonzentrier-Membran zu einer direkt beheizbaren Komposit-Membran zusammengefaßt sind. Dabei kann die Komposit-Membran ein elektrisch leitendes Stützgerüst aufweisen, auf dem die eigentliche selektive Schicht der Aufkonzentrier-Membran aufgebracht ist.

Die Wirtschaftlichkeit läßt sich weiterhin dadurch erhöhen, daß die aus dem Endpermeat- bzw. Endretentatbereich der Aufkonzentrier-Membran austretenden Dampfströme durch Wärmetauscher strömen, durch die andererseits das Fluidgemisch strömt. Damit wird ein Teil der dem Fluidgemisch zuzuführenden Umwandlungswärme, die für das Hindurchtreten der dampfförmigen Komponenten benötigt wird, durch die Kondensation der "Endprodukte" wieder aufgebracht bzw. zurückgewonnen.

Durch die bisher beschriebenen Maßnahmen wird eine hohe Aufkonzentrierung der Lösungsmittel um einen Faktor von ungefähr 10 bis 20 erreicht. Es ist beispielsweise mit der lösemittelselektiven Pervaporationsmembran aus Polydimethylsiloxan möglich, aus einem in einem Bioreaktor anfallenden Fluidgemisch, das 6% Äthanol enthält, einen Dampfstrom mit einer Äthanol-Konzentration von 40% zu gewinnen. Durch die Aufkonzentrier-Membran wird die Äthanol-Konzentration auf 80 bis 90% erhöht.

Für manche Anwendungsfälle - beispielsweise für die Gewinnung von Äthanol als Kraftstoff - ist jedoch ein Wasser-/Äthanol-Gemisch mit einer Äthanol-Konzentration von 80 bis 90% noch nicht ausreichend.

Für eine solche Konzentration ist es von Vorteil, wenn das dampfförmige Endretentat vor dem Wärmeaustausch mit dem Fluidgemisch über ein für wenigstens eine zusätzliche Komponente selektives Absorbens geleitet wird. Wenn die abzutrennende Komponente ein organisches Lösemittel, insbesondere Äthanol, ist und die zusätzliche Komponente Wasser ist, ist es von Vorteil, wenn das Endretentat dampfförmig über ein Absorbens für Wasser, insbesondere über gebrochenen Weizen, geleitet wird.

Das erfindungsgemäße Verfahren und die erfindungsgemäße Vorrichtung zum Abtrennen von Komponenten aus einem Fluidgemisch kann für sämtliche Anwendungsfälle eingesetzt werden, bei denen aus einem Fluidgemisch ein oder mehrere Komponenten, beispielsweise Lösemittel, abgetrennt werden sollen.

Besonders vorteilhaft ist jedoch die Verwendung des erfin dungsgemäßen Verfahrens sowie der erfindungsgemäßen Vorrichtung zum kontinuierlichen Abtrennen von Lösemitteln aus einem Bioreaktor, indem diese unter Verwendung von nachwachsenden Rohstoffen, wie Zuckerrüben, Kartoffeln oder Getreide sowie zucker- oder stärkehaltigen Industrieabfällen erzeugt werden. Es ist möglich, die bisher übliche diskontinuierliche Produktion durch eine kontinuierliche Produktionsweise zu ersetzen. Dies führt zu einer wesentlichen Senkung der Herstellungskosten.

Darüber hinaus wird die Effizienz der Bioproduktion durch die ständige Konzentrationssteuerung von reaktions-inhibierenden Substanzen gesteigert.

Das erfindungsgemäße Verfahren und die erfindungsgemäße Vorrichtung sind bzw. arbeiten besonders dann wirtschaftlich, wenn die Fluidgemischzuführung und die Retentatabführung mit einem lösemittelerzeugenden Bioreaktor in Verbindung stehen, so daß das Fluidgemisch dem lösemittelerzeugenden Bioreaktor entnommen wird und das Retentat der Selektier-Membran dem Bioreaktor zugeführt wird. Vorteilhafterweise wird auch das Endpermeat dem Bioreaktor über ein Stellventil zugeführt.

Ein Ausführungsbeispiel der Erfindung wird nachstehend anhand einer Zeichnung näher erläutert, die eine Vorrichtung zum Abtrennen von wenigstens einer Komponente aus einem Fluidgemisch zeigt.

Die gezeigte Vorrichtung dient zum Abtrennen und Konzentrieren eines in einem Bioreaktor hergestellten Lösemittels, beispielsweise Äthanol.

Zur kontinuierlichen Produktion von Lösemitteln wird einem nicht dargestellten Bioreaktor ein Fluidgemisch entnommen, das als Komponente A ein Lösemittel mit einer Konzentration $X_1$, als Komponente B Wasser sowie eine Reihe weiterer Komponenten, wie beispielsweise $CO_2$, $O_2$, Nebenprodukte, Nährsalz, Substrate, Mikroorganismen etc. enthält, die pauschal mit C bezeichnet sind.

Die Konzentration der Lösemittel-Komponente A in dem Fluidgemisch beträgt ungefähr 6%.

Das Fluidgemisch wird einem Pervaporations-Membranmodul 1 zugeführt, das eine erste Trennstufe bildet. Das Pervaporations-Membranmodul 1 ist in an sich bekannter Weise ausgebildet, wie dies beispielsweise in der PCT-Veröffentlichung WO 86/01425 beschrieben ist. Das Pervaporations-Membranmodul 1 weist eine Selektier-Membran 2 auf, die aus zwei Schichten, einer selektiven Schicht und einer porösen Schicht besteht. Die selektive Schicht der Selektier-Membran 2 ist ein Elastomer, dessen Permeabilität mit steigendem Molekulargewicht des Permeanten zunächst zunimmt. Damit ist die Permeabilität der Selektiermembran 2 für Lösemittel größer als für Wasser (Komponenten mit sehr hohem Molekulargewicht können bei dieser Betrachtung außer Betracht bleiben, da die genannten Gesetzmäßigkeiten nur bei vergleichsweisen niedrigen Molekulargewichten gelten). Die selektive Schicht führt als homogener Polymerfilm die eigentliche Stofftrennung durch, während die poröse Schicht den zwangsläufig sehr dünnen und empfindlichen homogenen Polymerfilm bei vernachlässigbarem Stoffwiderstand mechanisch verstärkt. Die selektive Schicht besteht aus Polydimethylsiloxan. Eine derartige Schicht besitzt eine hohe Permeabilität für Äthanol, $CO_2$ und andere niedermolekulare organische Lösungsmittel. Ferner weist sie auch eine gewisse Durchlässigkeit für $H_2O$ und $O_2$ auf. Die poröse Trägerstruktur, die die Permeation der einzelnen Stoffe vernachlässigbar gering hemmt, besteht aus einer porösen Polysulfon-Struktur.

Bei Verwendung einer derartigen Selektier-Membran 2 erhält man bei einer eintrittsseitigen Konzentration $X_1$ = 6% des Lösemittels A eine Konzentration $X_2$ von 40% im Permeatbereich $1'$ des Pervaporations-Membranmoduls 1. Ferner ist in dem Permeatbereich $1'$ noch die Komponente B, also Wasser, vorhanden.

Das Retentat des Pervaporationsmembran-Moduls enthält weiterhin die Komponenten A, B und C, wobei allerdings die Komponente A mit einer Konzentration $X_5$ vorliegt, die wesentlich kleiner als die eintrittsseitige Konzentration $X_1$ ist.

Das Permeat wird aus dem Permeatbereich $1'$ des Pervaporationsmembran-Moduls 1 mittels einer Vakuumpumpe 4 abgezogen, in einem Wärmetauscher 5 weiter erwärmt und tritt in ein zweites Aufkonzentrier-Membranmodul 6 ein, das aus einer Reihe von Kammern besteht und eine Aufkonzentrier-Membran 7 mit einer zur Selektivität der Selektier-Membran 2 inversen Selektivität aufweist, die das Aufkonzentrier-Membranmodul in einen Endretentatbereich 11 und einen Endpermeatbereich trennt.

Die selektive Schicht der Aufkonzentrier-Membran 7 ist dagegen ein glasartiges Polymer, dessen Permeabilität mit dem Molekulargewicht der permeierenden Komponente zunimmt.

Damit tritt durch die Aufkonzentrier-Membran 7 bevorzugt die Komponente B, also Wasser, durch. In dem Endretentatbereich 11 des Aufkonzentrier-Membranmoduls 6 wird die Komponente A, also das oder die Lösemittel, weiter angereichert. Auf diese Weise erhält man bei einer eintrittsseitigen Konzentration $X_2$ des Lösemittels A von 40% eine austrittsseitige Konzentration $X_4$ des Lösemittels A in dem Endretentatbereich 11 von 80 bis 90%.

Das Endpermeat im Aufkonzentriermembran-Modul 6, in den die Komponente B, also Wasser mit einer vergleichsweise großen Konzentration $X_3$ vorliegt, wird mit einer Vakuumpumpe 8 in der dampfförmigen Phase abgezogen und gibt in einem Wärmeaustauscher 9 Wärme an das aus dem Bioreaktor entnommene Fluidgemisch ab, so daß dieses vor seinem Eintritt in das Pervaporations-Membranmodul 1 erwärmt wird.

Das durch den Wärmeaustausch kondensierte Endpermeat des Aufkonzentriermembran-Moduls 6, wird wieder dem (nicht dargestellten) Bioreaktor zugeführt.

Das aus dem Endretentatbereich 11 entnommene Endretentat des Aufkonzentriermembran-Moduls 6, in dem die Komponente A, also das oder die Lösemittel mit einer Konzentration $X_4$ von typischerweise 80 bis 90% vorliegen, wird ebenfalls dampfförmig über einen Wärmetauscher 10 geleitet, wobei es Wärme an das Fluidgemisch abgibt und kondensiert. Anschließend wird es einer weiteren Verarbeitung zugeführt.

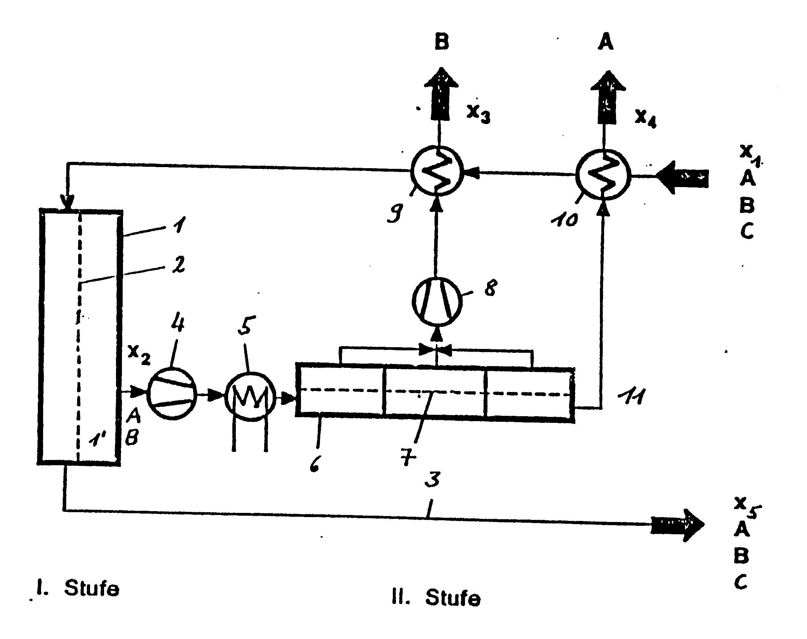

## Ansprüche

1. Verfahren zum Abtrennen wenigstens einer Komponente, insbesondere von Lösemitteln, aus einem Fluidgemisch, bei dem das Fluidgemisch an einer Selektier-Membran vorbeigeleitet und an dieser durch einen permeatseitigen Partialdruck, der niedriger ist als der des Fluidgemischs, in ein Permeat, dessen Konzentration an abzutrennender Komponente höher ist als die des Fluidgemischs, und in ein Retentat getrennt wird, dessen Konzentration an abzutrennender Komponente geringer ist als die des Fluidgemischs, und bei dem wenigstens das Permeat aufgeheizt wird, dadurch **gekennzeichnet,**

daß das Permeat der Selektier-Membran an einer Aufkonzentrier-Membran mit einer bezüglich der Selektier-Membran inversen Selektivität vorbeigeleitet wird, an der es durch einen endpermeatseitigen Partialdruck, der niedriger ist als der des vorbeigeleiteten Permeats, in ein Endpermeat, dessen Konzentration an abzutrennender Komponente geringer ist als die des Permeats der Selektier-Membran, und in ein Endretentat getrennt wird, dessen Konzentration an abzutrennender Komponente höher ist als die des Permeats der Selektier-Membran.

2. Verfahren nach Anspruch 1, dadurch **gekennzeichnet,** daß das Permeat der Selektier-Membran dampfförmig ist.

3. Verfahren nach Anspruch 1 oder 2, dadurch **gekennzeichnet,** daß das Permeat der Selektier-Membran zwischen den beiden Membranen so aufgeheizt wird, daß es dampfförmig an der Aufkonzentrier-Membran vorbeigeleitet wird.

4. Verfahren nach Anspruch 3, dadurch **gekennzeichnet,** daß das Permeat der Selektier-Membran durch die Aufkonzentrier-Membran aufgeheizt wird.

5. Verfahren nach einem der Ansprüche 1 bis 4, dadurch **gekennzeichnet,** daß das Endpermeat und das Endretentat dampfförmig in Wärmeaustausch mit dem Fluidgemisch gebracht werden.

6. Verfahren nach Anspruch 5, dadurch **gekennzeichnet,** daß das dampfförmige Endretentat vor dem Wärmeaustausch mit dem Fluidgemisch über ein für wenigstens eine zusätzliche Komponente selektives Absorbens geleitet wird.

7. Verfahren nach einem der Ansprüche 1 bis 6, dadurch **gekennzeichnet,** daß die abzutrennende Komponente ein organisches Lösemittel, insbesondere Äthanol, und die zusätzliche Komponente Wasser ist.

8. Verfahren nach Anspruch 7, dadurch **gekennzeichnet,** daß das Endretentat dampfförmig über ein Absorbens für Wasser, insbesondere über gebrochenen Weizen, geleitet wird.

9. Verfahren nach einem der Ansprüche 1 bis 8, dadurch **gekennzeichnet,** daß das Fluidgemisch einem lösemittelerzeugenden Bioreaktor entnommen wird und daß das Retentat der Selektier-Membran dem Bioreaktor zugeführt wird.

10. Verfahren nach Anspruch 9, dadurch **gekennzeichnet,** daß das Endpermeat dem Bioreaktor zugeführt wird.

11. Vorrichtung zum Abtrennen von wenigstens einer Komponente, insbesondere von Lösungsmitteln, aus einem Fluidgemisch
- mit einem Membranmodul (1), das durch eine für die abzutrennenden Komponenten durchlässigen Selektier-Membran (2) in einen Permeatbereich (1´) und einen Retentatbereich getrennt ist, der mit einer Fluidgemischzuführung und einer Retentatabführung (3) in Verbindung steht,
- mit einer Pumpe (4), die einlaßseitig mit dem Permeatbereich (1') für ein Abziehen von Permeat verbunden ist, und
- mit einer Heizeinrichtung (5) für wenigstens das Permeat,
dadurch **gekennzeichnet,** daß die Pumpe (4) förderseitig mit einem Aufkonzentrier-Membranmodul (6) verbunden ist, das durch wenigstens eine bezüglich der Selektier-Membran (2) invers selektive Aufkonzentrier-Membran (7) in einen Endretentatbereich (11) und einen mit dem Einlaß einer weiteren Pumpe (8) verbundenen Endpermeatbereich (11) getrennt ist.

12. Vorrichtung nach Anspruch 11, dadurch **gekennzeichnet,** daß die Aufkonzentrier-Membran (7) eine Lösungs-Diffusionsmembran ist.

13. Vorrichtung nach Anspruch 11 oder 12, dadurch **gekennzeichnet,** daß die Selektier-Membran (2) eine Pervaporations-Membran ist.

14. Vorrichtung nach einem der Ansprüche 11 bis 16, dadurch **gekennzeichnet,** daß die Selektier-Membran (2) eine selektive Schicht aus einem Elastomer und die Aufkonzentrier-Membran (7) eine selektive Schicht aus einem glasartigen Polymer aufweist.

15. Vorrichtung nach einem der Ansprüche 11 bis 13, dadurch **gekennzeichnet,** daß die Heizeinrichtung (5) zwischen dem Membranmodul (1) und der Aufkonzentrier-Membran (7) vorgesehen ist.

16. Vorrichtung nach einem der Ansprüche 11 bis 13, dadurch **gekennzeichnet,** daß die Heizeinrichtung (5) und die invers selektive Membran (7) zu einer direkt beheizbaren Kompositmembran zusammengefaßt sind.

17. Vorrichtung nach Anspruch 15, dadurch **gekennzeichnet,** daß die Kompositmembran ein elektrisch leitendes Stützgerüst aufweist, auf dem die Aufkonzentrier-Membran (7) aufgebracht ist.

18. Vorrichtung nach einem der Ansprüche 11 bis 17, dadurch **gekennzeichnet,** daß der Endpermeatbereich und der Endretentatbereich (11) jeweils mit einem Wärmetauscher (9, 10) in Verbindung stehen, durch den die Fluidzuführung läuft.

19. Vorrichtung nach Anspruch 18, dadurch **gekennzeichnet,** daß zwischen dem Endretentatbereich (11) und dem mit diesem verbundenen Wärmetauscher (10) ein für wenigstens eine zusätzliche Komponente selektives Absorbens angeordnet ist.

20. Vorrichtung nach Anspruch 19, dadurch **gekennzeichnet,** daß ein Absorbens für Wasser, insbesondere gebrochener Weizen, angeordnet ist, wenn die abzutrennende Komponente ein organisches Lösungsmittel, insbesondere Äthanol, und die zusätzliche Komponente Wasser ist.

21. Vorrichtung nach einem der Ansprüche 11 bis 20, dadurch **gekennzeichnet,** daß die Fluidgemischzuführung und die Retentatabführung (3) mit einem lösemittelerzeugenden Bioreaktor in Verbindung stehen.

22. Vorrichtung nach Anspruch 21, dadurch **gekennzeichnet,** daß der Endpermeatbereich über ein Stellventil mit dem Bioreaktor verbunden ist.

B      A

$X_3$     $X_4$

$X_1$
A
B
C

9    10

1

2

8

$X_2$   4    5

11

A
B

1'

6    7    3

$X_5$
A
B
C

I. Stufe       II. Stufe

EP 0 384 016 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 062 494 (KURARAY CO. LTD) * Zusammenfassung; Patentanprüche 1,5-7,9-11,14,16,17; Figuren 1,3; Seite 21, Zeilen 1-5; Seite 22, Zeilen 10-21; Seite 23, Zeilen 6-10; Seite 24, Zeilen 1-16; Seite 24, Zeile 27 - Seite 25, Zeile 16 * --- | 1-3,7, 11-15 | B 01 D 61/36 B 01 D 61/58 B 01 D 53/22 C 07 C 29/76 // C 12 M 1/12 |
| Y | CHEMIE INGENIEUR TECHNIK, Band 60, Nr. 8, August 1988, Seiten 590-603, Weinheim, DE; H. STRATHMANN et al.: "Die Entwicklung von lösungsmittelselektiven Membranen und ihre Anwendung in der Gastrennung und Pervaporation" * Seite 595, Spalte 2, Zeilen 52-59; Seite 602, Spalte 2, Zeile 14 - Seite 603, Spalte 1, Zeile 4; Figur 18 * --- | 1,2,7,9 -14,21 | |
| A | IDEM --- | 22 | |
| D,Y | WO-A-8 601 425 (FRAUNHOFER GES.) * Zusammenfassung; Patentanspüche 1,5,9,14; Seite 5, Zeilen 1-18; Seite 8, Zeilen 7-22; Seite 9, Zeilen 15-21; Figuren 2,4 * | 1,2,7,9 -14,21 | **RECHERCHIERTE SACHGEBIETE (Int. Cl.5)** B 01 D C 12 M C 12 G |
| A | --- -/- | 3,4,16, 17 | |

Der vorliegende Recherchenbericht wurde für alle Patentansprüche erstellt

| Recherchenort | Abschlußdatum der Recherche | Prüfer |
|---|---|---|
| DEN HAAG | 09-03-1990 | HOORNAERT P.G.R.J. |

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | PATENT ABSTRACTS OF JAPAN, Band 12, Nr. 92 (C-483)[2939], 25. März 1988; & JP-A-62 225 207 (AGENCY OF IND. SCIENCE & TECHNOL.) & DATABASE WPIL, Nr. AN-87/317502, Derwent Publ., London, GB, & CHEMICAL ABSTRACTS, Band 108, Nr. 12, 21. März 1988, Seite 151, Zusammenfassung Nr. 97062f; KASIWAGI TORU et al.: "Two-stage pervaporation process for separation of volatile organic liquid and water" * Zusammenfassung * --- | 1,2,7, 11-13 | |
| A | IDEM --- | 3,14 | |
| D,Y | US-A-3 608 610 (J.L. GREATOREK) * Zusammenfassung; Patentanspruch 1; Figuren 1-4; Spalte 2, Zeilen 19-31 * | 1,2,7, 11-13 | |
| A | --- | 3,4,16, 17 | |
| A | JOURNAL OF MEMBRANE SCIENCE, Band 16, Nr. 1-3, Dezember 1983, Seiten 269-284, Amsterdam, NL; M.H.V. MULDER et al.: "Ethanol-Water Separation by Pervaporation" * Zusammenfassung; Seite 269, Zeile 1 - Seite 271, Zeile 16; Seite 275, letzter Absatz - Seite 277, Zeile 25; Figur 1 * --- | 1,2,7,9 -14,21, 22 | RECHERCHIERTE SACHGEBIETE (Int. Cl.5) |
| A | EP-A-0 029 600 (H. MICHELE) * Zusammenfassung; Patentansprüche 1,10-12; Figur 7; Seite 8, Zeile 8 - Seite 9, Zeile 18; Seite 18, Zeile 23 - Seite 20, Zeile 26; Seite 22, Zeilen 1-8 * --- -/- | 1,2,7, 11,13 | |

Der vorliegende Recherchenbericht wurde für alle Patentansprüche erstellt

| Recherchenort | Abschlußdatum der Recherche | Prüfer |
|---|---|---|
| DEN HAAG | 09-03-1990 | HOORNAERT P.G.R.J. |

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 240 803  (GKSS) <br> * Figuren 1,2; Zusammenfassung; Patentansprüche 1-3; Seite 2, Zeile 29 - Seite 7, Zeile 12 * <br> ----- | 1,2,5,7 ,9-14, 18,21, 22 | |

RECHERCHIERTE
SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| DEN HAAG | 09-03-1990 | HOORNAERT P.G.R.J. |

EPO FORM 1503 03.82 (P0403)